# EUROPEAN PATENT APPLICATION

(11) **EP 3 205 319 A1**
(43) Date of publication of application: **16.08.2017**
(21) Application number: 17152663.5
(22) Date of filing: 23.01.2017
(51) Int. Cl.: A61F 13/551, A47D 5/00, A47D 15/00, A45C 7/00, A61F 13/15

(54) **HYGIENIC DIAPER CHANGER**

(30) Priority: 11.02.2016 PT 2016109151
(71) Applicant: Ovalle-Bahamon, Ricardo Edgard, 0000 Luanda (AO)
(72) Inventor: Ovalle-Bahamon, Ricardo Edgard, 0000 Luanda (AO)
(74) Representative: Pereira da Cruz, Joao

(57) **Abstract**

The present invention addresses a hygienic diaper changer. The diaper changer, object of the present invention is constituted by a bag that has an opening (1) at the top of the bag, through which the baby is inserted feet first to the waist and has lateral access openings (2) through which are inserted the hands of the caregiver, allowing in this manner the easy changing of the diaper, in addition there is a central opening (3) to facilitate the complete process of changing the diaper without soiling the surface where the activity is taking place and preventing the propagation of odors. When placed on the child, the diaper changer seals, preventing the passing of odors or fluids to the exterior.

## Description

### Technical field of the invention

The present invention is in regards to a hygienic diaper changer, inserted within the technical area of child care, and framed within the class A61F13/49, of the International Classification for Patents.

### Background of the invention

In today's world it is ever more useful to find products that facilitate the dislocation of parents in the company of their babies, especially since these are completely dependent on their progenitors. There are numerous products in the market that facilitate the dynamics in external environments for families with small children, from baby carts, backpacks to transport the small ones, multi-purpose bags with many organizing pockets and the diaper changers.

There are many diaper changers in the market today, in the majority these are "blankets" with fabric on one side and the other side absorbent or impermeable material, they are padded in most cases to make them more comfortable for the babies with coverage for the child from head to the area where the diaper is changed. These products are very useful when we wish to change the diaper outside the home, needing only to have a flat surface where we can lay the child.

A problem associated with these diaper changers appears when we find ourselves inside various forms of transportation, for example, a bus or car, and especially an airplane and we need to change a diaper while in movement, or even when stopping the vehicle, but it is not possible to realize these tasks. It is very common when changing a diaper on a plane that the problem of the odor comes up, or that the diaper changer does not prevent the surface where it is placed from stains. These problems also surface in situations where we are standing still, but in fact they are more evident in the situations described.

To avoid these situations rises the present invention, which is a diaper changer in the form of a bag, where the child is inserted up to the waist and it has openings on the sides for the hands of the caregiver so that he/she can change the diaper without running the risk of soiling the surface where the operation is implemented, and preventing the propagation of odors, given that once the diaper changer is placed on the baby it seals the odors, not allowing the passing of odors or fluids to the exterior. We should add that all the materials of the diaper changer are flexible, allowing easy access and handling of its interior, and after its use it is removed from the baby, leaving the soiled diaper in its interior, closing the whole diaper changer to be properly disposed.

The present invention presents various advantages in relation to the current level of technology (state of art):
- It is simple and flexible, allowing the changing of diapers in any environment, in or out of the house, and in any form of transportation;
- It can be used in restricted spaces (as in seats of buses or planes) to avoid odors from bothering people near the babies and their caregivers;
- It can be used outdoors, where the environment can present a threat to hygienic conditions for the baby;
- It is small and light, safe and secure, comfortable and easy to use;
- Allows easy access for the baby to a completely safe environment;
- It is projected for use in planes, long distance travels, or open air;
- Allows coverage of the baby from the waist down, allowing comfort without restricting breathing;
- Removal of the changer allows for all waste to remain within its interior preventing the propagation of odors.

### Description of the Invention

The present invention addresses a hygienic diaper changer. The diaper changer, object of the present invention is constituted by a bag with an opening on one end (1), through which the baby/child is inserted feet first, up to the waist. This opening (1) is sealed by a thread (4) to assure the baby is secure without causing discomfort by pressure from an elastic.

In addition to the top opening (1), the diaper changing presents two lateral openings (2), through which are inserted the hands of the caregiver, allowing the changing of the diaper, and it also has a central opening (3), on the front of the diaper changer, to facilitate the total process of changing the diaper. The three openings (2) (3) consist of cross-cuts 90mm in length, with tabs (covers), preferentially circular tabs, attached to the interior of the openings, but separate to the diaper changer to allow access and to maintain odors in the interior of the station.

Each of these three openings (2) (3) can have a cover (tongue) that closes automatically to avoid odors from escaping and to avoid dust or other pollutants that may be harmful to the baby from entering the diaper changer.

At the bottom of the diaper changer, more specifically, where the feet of the baby are placed, there is an interior pocket (6) to store the soiled diaper and other waste products to maintain the hygienic conditions within the recipient and to avoid that these bother the child or the caregiver during the changing of the diaper.

At lower part of the diaper changer there is also a flexible support ring (7) that allows folding of the recipient at the same time it provides vertical support that facilitates changing of the diaper.

The back of the diaper changer, where the back of the baby will lie, has a foam covering (8) (with a thickness preferably between 0.5mm and 2.0mm) or rubber (with a thickness preferably between 1mm and 2mm) to protect the baby and increase comfort.

All surfaces of the diaper changer are made of impermeable plastic and are malleable so that it can be folded to store and comfortable to use. To reduce visibility for people nearby in the immediate surroundings, the side panels and bottom of the diaper changer are made in colored and not translucent plastic. The top (front) part of the diaper changer is made of transparent plastic so that the person changing the diaper can easily see the baby and the changing of the diaper.

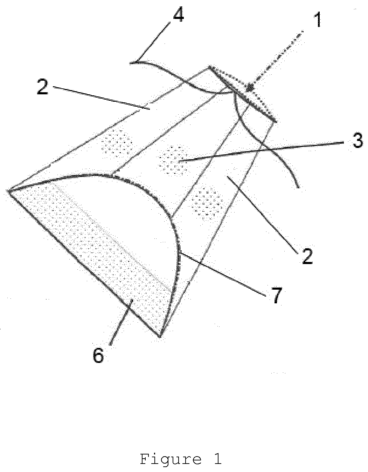

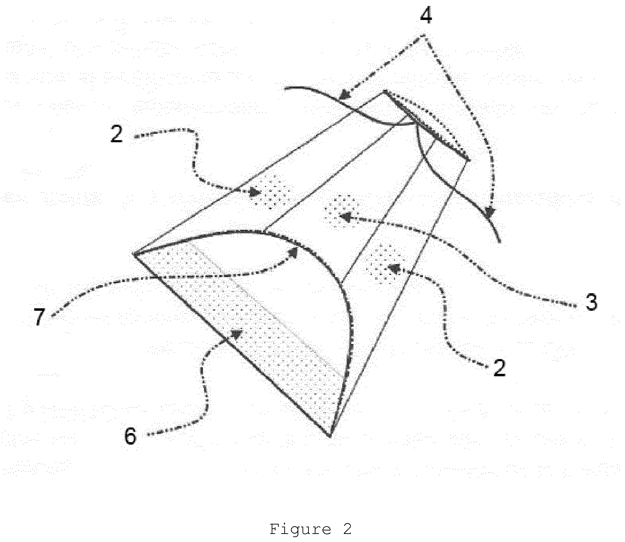

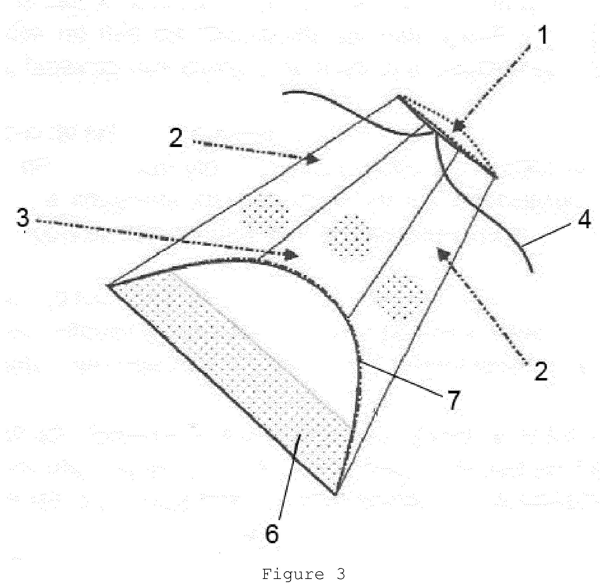

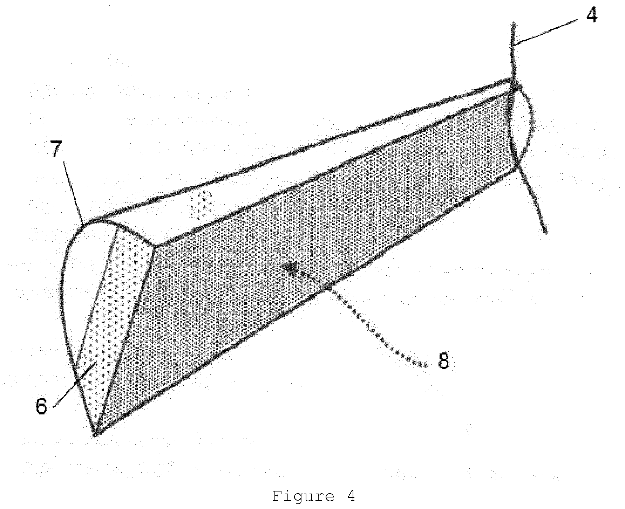

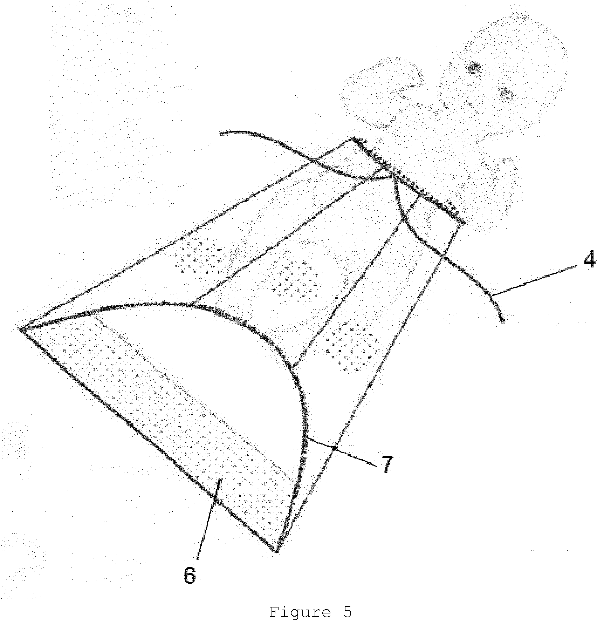

## Claims

1. Hygienic diaper changer **characterized in that** it is a bag made of flexible material comprising:
a) opening at the top (1) sealed by a thread (4);
b) lateral openings (2);
c) central opening (3) at the front of the diaper changer;
d) flexible support ring (7) at the lower part;
e) covering (8) at the back where the child is lying;
f) interior pocket (6) at the bottom part.

2. Hygienic diaper changer according to claim 1 wherein the openings (2) (3) are crisscross cuts 90mm in length.

3. Hygienic diaper changer according to claim 1 wherein the openings (2) (3) have covering tabs in the interior of the opening but exterior to the diaper changer.

4. Hygienic diaper changer according to claim 1 wherein the openings (2) (3) have a covering that closes automatically.

5. Hygienic diaper changer according to the previous claims wherein the material of the covering (8) is foam and the thickness between 0,5mm and 2,0mm.

6. Hygienic diaper changer according to the previous claims wherein the material of the covering (8) is rubber and the thickness between 1mm and 2mm.

7. Hygienic diaper changer according to the previous claims wherein its surfaces are made of flexible and impermeable plastic.

8. Hygienic diaper changer according to the previous claims wherein its side and bottom surfaces are made of color and not translucent plastic.

9. Hygienic diaper changer according to the previous claims wherein the front surface is made of transparent plastic.
